(19) Europäisches Patentamt / European Patent Office / Office européen des brevets

(11) **EP 2 573 557 A1**

(12) **EUROPEAN PATENT APPLICATION**
published in accordance with Art. 153(4) EPC

(43) Veröffentlichungstag:
27.03.2013  Patentblatt 2013/13

(21) Anmeldenummer: 11783811.0

(22) Anmeldetag: 28.02.2011

(51) Int Cl.:
*G01N 33/38* (2006.01)    *G01N 3/00* (2006.01)

(86) Internationale Anmeldenummer:
PCT/RU2011/000112

(87) Internationale Veröffentlichungsnummer:
WO 2011/145977 (24.11.2011 Gazette 2011/47)

(84) Benannte Vertragsstaaten:
AL AT BE BG CH CY CZ DE DK EE ES FI FR GB GR HR HU IE IS IT LI LT LU LV MC MK MT NL NO PL PT RO RS SE SI SK SM TR

(30) Priorität: 18.05.2010  RU 2010119593

(71) Anmelder: Anpilov, Sergej Mikhajlovich
Samarskaya obl. 446960 (RU)

(72) Erfinder:
• VOLKOV, Jurij Veniaminovich
Samara 443076 (RU)

• MURASHKIN, Gennadiy Vasilievich
Samara 443096 (RU)
• MURASHKIN, Vasiliy Gennadievich
Samara 443096 (RU)
• RYZHKOV, Andrej Sergejevich
Samarskaja obl. 445001 (RU)

(74) Vertreter: Jeck, Anton
Jeck - Fleck - Herrmann
Klingengasse 2/1
71657 Vaihingen/Enz (DE)

(54) **VERFAHREN UND VORRICHTUNG ZUR ANALYSE DER STRUKTUR UND DER STÄRKE VON BETON**

(57)    Die Erfindung ist im Bauwesen und zwar auf ein Verfahren und eine Einrichtung zur Betongefügeanalyse und Betonfestigkeitsprüfung einsetzbar. Das Verfahren umfasst ein Schleifen der zu untersuchenden Stelle der Oberfläche des Betonerzeugnisses und eine Ermittlung ihrer Oberflächenfestigkeit im Trockenzustand, das Einweichen dieser Stelle und die Ermittlung ihrer Oberflächennassfestigkeit, das Bohren von Beton und die Messung von Leistung, Weggröße und Geschwindigkeit des Bohrwerkzeugs mit Ausgabe der Messdaten in Form von Kennlinien und die Ermittlung des lagenweisen Gefüges und der lagenweisen Nassfestigkeit, die Entnahme von einer zylinderförmigen Referenzprobe, ihre Analyse in Bezug auf Druck- oder axiale Zugfestigkeit und den Vergleich der Angaben mit den anhand anderer Verfahren ermittelten Angaben. Die Erfindung ermöglicht es, die Genauigkeit der Betongefügeanalyse und der Betonfestigkeitsprüfung bei Bauteilen zu erhöhen.

EP 2 573 557 A1

**Beschreibung**

Die Erfindung betrifft ein Verfahren zur Betongefügeanalyse und Betonfestigkeitsprüfung bei Bauteilen nach dem Oberbegriff des Anspruchs 1 und eine Einrichtung zur Durchführung des Verfahrens.

Die Erfindung ist im Bauwesen und zwar beim Bau und Betrieb von Gebäuden und anderen Bauwerken einsetzbar. Die Erfindung betrifft insbesondere die Forschung von Festigkeitseigenschaften von Material und zwar die Betongefügeanalyse und die Betonfestigkeitsprüfung. Sie kann zur Festigkeitsprüfung von solchen Betonen angewendet werden, die in verschiedenen Bauten und Erzeugnissen bei der Fertigung, dem Bau, der Untersuchung und der Prüfung sowie bei der Betriebsüberwachung und Kontrolle des Zustands von Bauwerken nach ihrem Dauerbetrieb verwendet werden.

Durch das Patent RU 2235322, KI. G 01 N 33/38, 2004, ist ein Verfahren bekannt, welches die Ermittlung und den Vergleich von verschiedenen Kenngrößen der Betonproben einschließt. Diese Betonproben kennzeichnen ihre physikalischen Eigenschaften nach der Trocknung in einer Trockenluftumgebung und unter kompletter Wassersättigung. Die Zustandsprüfung wird anhand des Risswiderstand-Kennwerts vorgenommen. Als Kenndaten werden die Grenzflächenenergien benutzt, die nach der Trocknung bis zur Verfestigung sowie im kompletten Wassersättigungszustand unter Umgebungsluftdruck und bei gleicher Betonzusammensetzung gemessen werden. Die Grenzflächenenergie wird ermittelt, indem die Ausbohrungszeiten in den Proben verglichen werden, vorausgesetzt, dass die Kenndaten der Ausbohrungen - Durchmesser und Tiefe - in der zu prüfenden Probe und in der Referenzprobe mit bekannter Grenzflächenenergie gleich sind.

Die Nachteile dieses bekannten Verfahrens sind wie folgt: Es gibt keine Vorbehandlung der zu untersuchenden Oberfläche. Das entstellt die Prüfungsergebnisse. Darüber hinaus ermöglicht das vorgenommene Ausbohren der Betonproben nicht, die lagenweisen Kenndaten für den Betonzustand zu bekommen. Es ist auch nicht möglich, eine Referenzbetonprobe zu bekommen.

Aus dem Patent RU 3452179722, KI. G 01 N 33/38, 2002, ist ein Verfahren zur Analyse des Betongefüges und der Betonfestigkeit bekannt, das als Prototyp angesehen wird. Es umfasst ein Bohren des Betons mit Hilfe einer Diamantbohranlage und die Messung der Betonoberflächenfestigkeit. Die Probestelle in dem zu messenden Beton wird vor der Prüfung für eine Zeit lang eingeweicht. Die Probe wird mit Hilfe eines Bohrwerkzeugs entnommen. Die Oberflächenfestigkeit wird unter Berücksichtigung der Feuchtigkeit der jeweiligen Stelle ermittelt. Die Bohranlage wird auf diese Stelle aufgestellt und gestartet. Die Belastung des Bohrwerkzeugs wird konstant aufrechterhalten. Während der Probenahme werden gleichzeitig die Bohrleistung sowie die Größe und die Geschwindigkeit des Vorschubs des Bohrwerkzeugs gemessen. Diese Daten werden mittels EDV ausgewertet, indem die Messdaten mit der bekannten gemessenen Betonoberflächenfestigkeit verglichen werden. Die bekannte Betonoberflächenfestigkeit wurde vorher im Rechner abgespeichert. Danach werden die Daten in Form von Leistungs- und Vorschubkennlinien, Geschwindigkeitskennlinien des Bohrwerkzeugs ausgegeben. Diese Kennlinien kennzeichnen das Gefüge und die lagenweise Festigkeit des Betons auf einem Rechner-Bildschirm. Dabei werden auch die digitalen Daten für jede Kennlinie mit ausgegeben.

Jedoch werden die Betonprüfungen auf einer unvorbereiteten Oberfläche durchgeführt. Diese Oberfläche kann Unebenheiten bzw. herausragende Steinschotter und Bewehrung aufweisen. Darüber hinaus gibt es keine Möglichkeit, eine Referenzbetonprobe für weitere Prüfungen zu bekommen und die Festigkeit anhand der Referenzproben unter Laborbedingungen in Übereinstimmung mit GOST 18105-86, GOST 10180-90 zu ermitteln.

Ein durch das Erfinderzertifikat 1807392, KI. G 01 N 33/38, 1993, bekanntes Betonfestigkeitsmessgerät weist einen in einem Gehäuse angeordneten Schlagkörper und eine Rückprallsicherung auf. Das untere Ende des Schlagkörpers ist mittels einer Feder mit einem Schlagstück gekoppelt. Das obere Ende ist mit einer Schlagführungseinheit gekoppelt. Der Schlagkörper ist mit einem Anschlag versehen. Der Anschlag ist mit der Rückprallsicherung gekoppelt. Die Einrichtung ist mit einem Wegwandler versehen, der mit der Rückprallsicherung verbunden ist. Darüber hinaus ist die Einrichtung mit einem Sollwertgeber für einen Gehäuseneigungswinkel zur Untersuchungsoberfläche, einer Messuhr mit einer Einheit für einen Fehlerausgleich und einer Messbereichseinstellung, einem Maßstabteiler, drei Schaltern, einem Zeitschalter, einem Schlagzahl-Dezimalzähler, einem Impuls-Summenzählwerk, einer Anlasseinheit und einem Normierungsimpulsbilder versehen. Der Normierungsimpulsbilder ist in Form von nacheinander geschalteten Integrierer und Komparator ausgebildet. Der Eingang des Integrierers ist der Eingang des Normierungsimpulsbilders. Der Ausgang des Normierungsimpulsbilders ist der Ausgang des Komparators. Dabei ist der Ausgang des Wegwandlers über den Sollwertgeber des Neigungswinkels an die Messuhr und über einen Öffnerkontakt des ersten Schalters an den Eingang der Anlasseinheit angeschlossen. Ein Ausgang der Anlasseinheit ist an den Eingang des Zeitschalters und der andere Ausgang ist über den Maßstabteiler an den Eingang des Normierungsimpulsbilders angeschlossen. Der Ausgang des Normierungsimpulsbilders ist an den Eingang des zweiten Schalters angeschlossen. Der erste Schließerkontakt des zweiten Schalters ist in einen Rücksetzstromkreis des Integrierers des Normierungsimpulsbilders eingeschlossen. Der zweite Schließerkontakt des zweiten Schalters ist an den Zähleingang des Impuls-Summenzählwerks angeschlossen. Der Ausgang des Zeitschalters ist über den ersten Schalter an den Eingang des dritten Schalters angeschlossen, dessen Öffnerkontakt in den Stromversorgungskreis der Einrichtung eingeschlossen ist.

Diese Einrichtung ist etwas zu kompliziert aufgebaut und hat eine verwickelte Messschaltung mit einem Analogausgang.

Als Prototyp gilt die Einrichtung zur Analyse der Betonfestigkeit während der Probenahme aus Betonbauten mittels Probeausbohrungen (s. Patent RU 2198399, KI. G 01 N 33/38, 2003). Diese Einrichtung enthält eine Diamantbohranlage zum Bohren von Beton und ein Messgerät für eine Oberflächenbetonfestigkeitsmessung. Die Bohranlage ist mit einem Weggeber des Bohrwerkzeugs, einem Messgeber für Wirkleistung, die vom Antriebsmotor verbraucht wird, und einem Controller mit einem Rechner ausgerüstet. Dabei sind die Ausgänge des Weggebers und des Messgebers für die Wirkleistung an den Controller angeschlossen, der mit dem Rechner verbunden ist. Das Oberflächenfestigkeits-Messgerät dient zur Messung der Oberflächenbetonfestigkeit unter Beachtung seiner Feuchtigkeit, die im Rechner abgespeichert wird. Die Ausführung der Bohranlage ermöglicht es, sie auf der Stelle für die Oberflächenbetonfestigkeit aufzustellen und unter konstanter Belastung des Bohrwerkzeugs zu arbeiten. Die Leistungsmessgeber und die Weggeber dienen zur gleichzeitigen Messung der jeweiligen Größen während der Probenahme mittels Ausbohrung. Der Rechner dient zur EDV, indem die erfassten Daten unter Beachtung der gemessenen Oberflächenbetonfestigkeit verglichen werden. Die Daten werden als Leistungs-, Weg- und Bohrwerkzeuggeschwindigkeitskennlinien ausgegeben, welche die Betonfestigkeit kennzeichnen, sowie als eine Kennlinie, die die lagenweise Betonfestigkeit beschreibt.

Jedoch können bei einer Probenahme aus Betonbauten mit Hilfe dieser Einrichtung manche Messunsicherheiten wegen unvorbereiteter Oberfläche entstehen. Denn die unvorbereitete Oberfläche kann Unebenheiten aufweisen, die durch hervorragende Steinschotter und Bewehrung verursacht werden. Das bedingt, die Ergebnisse der Betongefügeanalyse und der Betonfestigkeitsprüfung zu entstellen. Darüber hinaus kann mit dieser Einrichtung keine Referenz- Betonprobe entnommen werden, um diese nachher für weitere Prüfungen gemäß GOST 18105-86, GOST 10180-90 benutzen zu können.

Es ist Aufgabe der Erfindung, ein Verfahren zu entwickeln, welches die Genauigkeit der Betongefügeanalyse und der Betonfestigkeitsprüfung der Bauteile erhöht und minimalen Arbeitsaufwand und minimale Kosten aufweist. Die weitere Aufgabe der Erfindung ist die Herstellung einer Einrichtung, welche es ermöglicht, genaue Ausgabedaten für die lagenweise Betongefügeanalyse des jeweiligen Erzeugnisses zu bekommen sowie eine zufriedenstellende Genauigkeit der Betonfestigkeitsmessung und -prüfung des Erzeugnisses dank der Vorbehandlung der zu untersuchenden Stelle der Oberfläche des Erzeugnisses zu erreichen. Des Weiteren soll eine Einrichtung entwickelt werden, welche es ermöglicht, während der Prüfung eine Referenz-Betonprobe zu entnehmen, um nachfolgend entsprechende Prüfungen durchzuführen und die Betonfestigkeit unter Laborbedingungen gemäß den Vorschriften von GOST 18105- 86, GOST 10180-90 zu ermitteln.

Die gestellten Aufgaben werden durch die Merkmale der Ansprüche 1 und 4 gelöst.

Das Verfahren wird wie folgt gelöst. Die zu untersuchende Stelle zur Ermittlung von Betongefüge und -festigkeit des Betonerzeugnisses wird ausgewählt. Die Oberfläche dieser Stelle wird vor der Prüfung vorbereitet. Dafür wird die zu untersuchende Stelle geschliffen. Die Oberflächenfestigkeit wird im Trockenzustand ermittelt. Danach wird diese Stelle der zur untersuchenden Oberfläche des Betonerzeugnisses eingeweicht. Die Betonoberflächenfestigkeit wird unter Beachtung seiner Feuchtigkeit bestimmt. Danach wird eine Bohranlage zum Betonausbohren auf die zu untersuchende Stelle aufgesetzt. Mittels Bohrens werden das lagenweise Gefüge und die lagenweise Betonnassfestigkeit ermittelt. Dabei wird infolge der Ausbohrung eine zusätzliche zylinderförmige Referenz-Betonprobe entnommen. Diese Referenzprobe wird für weitere Prüfungen bei der Ermittlung von Druck- oder axialer Zugfestigkeit der Referenz-Betonprobe verwendet. Dabei werden die Prüfungsmessdaten unter Laborbedingungen gemäß den Vorschriften von GOST 18105-86, GOST 10180-90 mit den Messdaten verglichen, welche nach den Verfahren ermittelt worden sind. Die Referenz- Betonprobe wird vorher getrocknet.

Um den Datenempfangsablauf zu steuern, werden die aktuellen Einstellungen der Geräte am Anfang des Bohrens angezeigt. Während des Bohrens werden die Momentanmessdaten der Kennwerte empfangen und über eine Datenübertragungsschnittstelle an einen PC oder an einen Microcontroller weitergeleitet zwecks Auswertung und Berechnung von Ergebnissen über den spezifischen Energieaufwand pro Vorschublängen-Einheit des Schneide-Bohrwerkzeugs während des Bohrens des Betonerzeugnisses und der Erstellung einer Leistungskennlinie und Vorschubkennlinie des Schneid-Bohrwerkzeugs.

Darüber hinaus wird die zylinderförmige Referenz-Betonprobe für weitere Prüfungen unter Laborbedingungen gemäß den Vorschriften von GOST 10180-90 hergestellt. Dabei wird für das Bohren des Betonerzeugnisses ein zylinderförmiges Hohl-Schneid-Bohrwerkzeug (Kernbohrer) mit vorgegebenem Innendurchmesser des Schneid-Bohrwerkzeugs gemäß Vorschriften von GOST 10180-90 verwendet. Darüber hinaus ist die Bohranlage der Einrichtung zur Betongefügeanalyse und Betonfestigkeitsprüfung der Bauteile mit einem Rechtwinkligkeitsregler für die Bewegung des Schneid-Bohrwerkzeugs, einem Druckgleichhalter, um den auf das Schneid-Bohrwerkzeug ausgeübten Druck konstant zu hatten, einem Messsignalwandler und einer Kühlschmiermittel-Rückführungsanlage ausgerüstet. Das Schneid-Bohrwerkzeug ist in Form eines Hohlzylinders ausgebildet. An einer Stirnfläche des Hohlzylinders sind ein Kupplungsstück, um ihn an den Vorschubantrieb anzuschlie-βen, und eine Kühlschmiermittel-Versorgungseinrichtung angebracht. An der anderen Stirnfläche sind Hartmetallschneidplatten befestigt.

Darüber hinaus sind die Hartmetallschneidplatten des Schneid-Bohrwerkzeugs so eingebaut, dass während des Bohrens eine zylinderförmige Referenz-Betonprobe mit dem Solldurchmesser entnehmbar ist. Dabei ist jede Hartmetallschneidplatte am Zylinder des Schneid-Bohrwerkzeugs symmetrisch befestigt, um die Messgenauigkeit der Kennwerte zu erhöhen, indem Messfehler je nach Körnung von Schotter und Betonzuschlagstoffen vermindert und ein Leistungsverlust der Bohranlage infolge der Reibung des Schneid-Bohrwerkzeugs an der Seitenfläche des Betonerzeugnisses vermieden werden. Die Breite jeder Hartmetallschneid-platte ist wenigstens um 1 mm größer als die Wandstärke des Zylinders des Schneid-Bohrwerkzeugs.

Darüber hinaus sind die Hartmetallschneidplatten am Zylinder des Schneid-Bohrwerkzeugs so angebracht, dass der vorgegebene Innendurchmesser für die Behandlung gebildet werden kann, der dem Außendurchmesser der Referenz-Betonprobe gemäß den Vorschriften von GOST 10180-90 oder gleich 100+0,1 mm oder 150+0,1 mm oder 200+0,1 mm oder 300+0,1 mm ist.

Darüber hinaus sind die Hartmetallschneidplatten so eingebaut, dass sie je nach Verschleiß ausgetauscht werden können.

Darüber hinaus ist der Messsignalwandler mit einem Leichtgängigkeitsregler mit einem Weg-Messwandler, einem Strom-Messwandler, einem Spannungs-Messwandler, einem Drehzahl-Messwandler, einem Umschalter, Schnittstelleneinheiten, Pufferverstärkern, einem A-D-Umsetzer, einer PC- oder Microcontroller-Schnittstelle ausgestattet. Dabei sind der Strom-Messwandler, der Spannungs-Messwandler und der Weg-Messwandler jeweils mit einem Pufferverstärker nacheinander geschaltet. Der Ausgang jedes Pufferverstärkers ist mit dem Eingang des Umschalters verbunden. Der Drehzahl-Messwandler ist in Reihe mit dem Pufferverstärker geschaltet, dessen Ausgang an den Eingang des Microcontrollers angeschlossen ist. Der Ausgang des Umschalters ist an den Eingang der Puffereinrichtung angeschlossen. Der Ausgang der Puffereinrichtung ist an den A-D-Umsetzer angeschlossen, dessen Ausgang mit dem zweiten Eingang des Microcontrollers verbunden ist. Ein Ausgang des Microcontrollers ist an den zweiten Eingang des Umschalters angeschlossen, und der andere Ausgang ist an den zweiten Eingang des A-D-Umsetzers angeschlossen. Der dritte Ausgang des Microcontrollers ist mittels Schnittstelleneinheiten über ein Filter mit dem PC in Reihe geschaltet.

Die Erfindung wird anhand der Zeichnungen näher erläutert. Es zeigen:

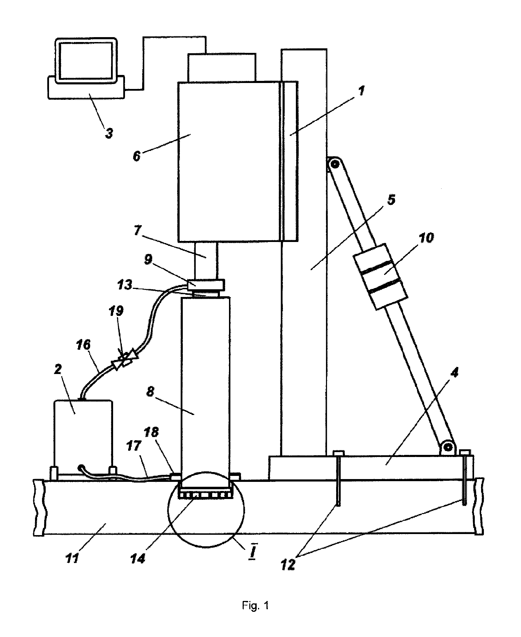

Fig. 1    einen Übersichtsschaltplan der Anlage zur Prüfung und Entnahme einer Referenz- Betonprobe,

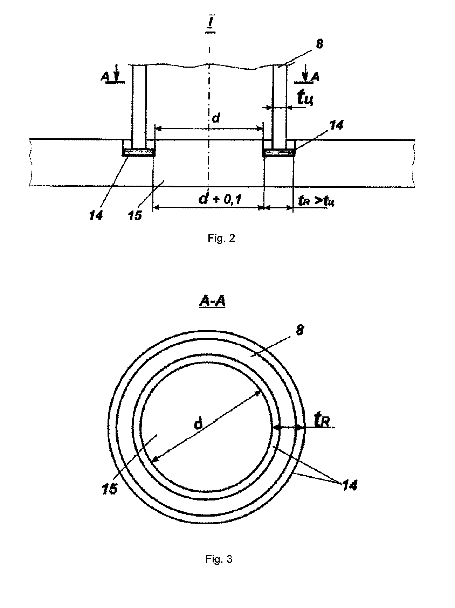

Fig. 2    die Baugruppe 1 aus Fig. 1 - Betonbohrbild und Entnahme der zylinderförmigen Betonprobe mittels eines zylinderförmigen Hohl-Schneid-Bohrwerkzeugs,

Fig. 3    den Schnitt A-A in Fig. 2 - zylinderförmiges Hohl-Schneid-Bohrwerkzeug (Kernbohrer) mit Hartmetallschneidplatten, die Hartmetallschneidplatten weisen auf den Solldurchmesser für eine Innenbehandlung des Betonerzeugnisses und für die Entnahme der zylinderförmigen Referenz-Betonprobe hin,

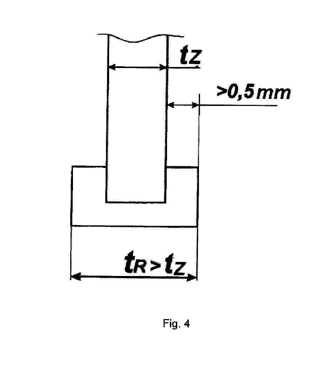

Fig. 4    die Anordnung der Hartmetallschneidplatte am zylinderförmigen Hohl-Schneid-Bohrwerkzeug,

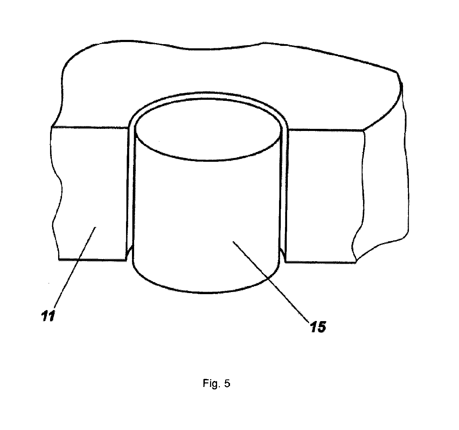

Fig. 5    die zylinderförmige Referenz-Betonprobe,

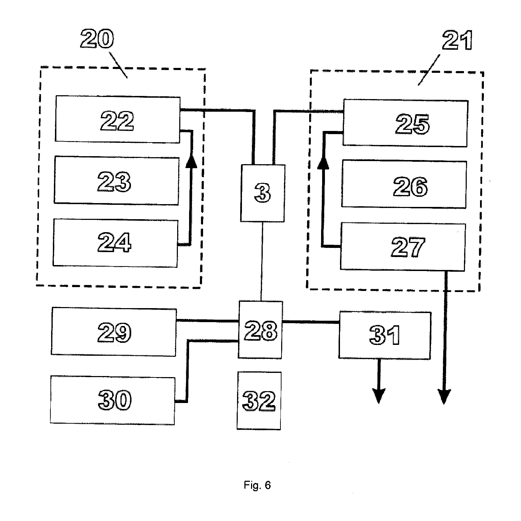

Fig. 6    ein Blockschaltbild der Einrichtung,

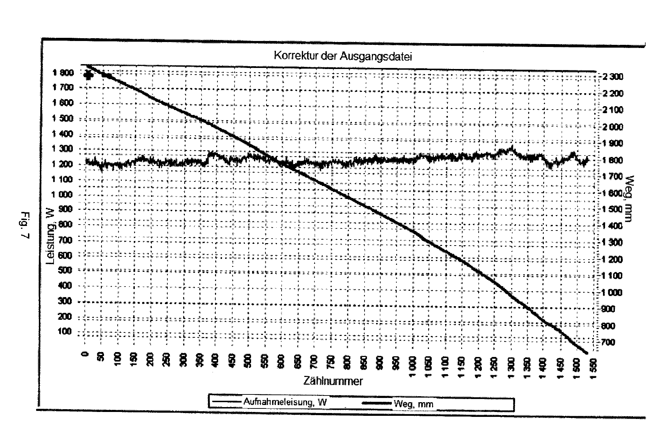

Fig. 7    die Weg-Kennlinie,

Fig. 8    die Energieaufwand-Kennlinie für eine Bohrung von 1 Millimeter im Material,

Fig. 9    die Abflachung der Kennlinie bei der Vergrößerung des Wertbereichs und

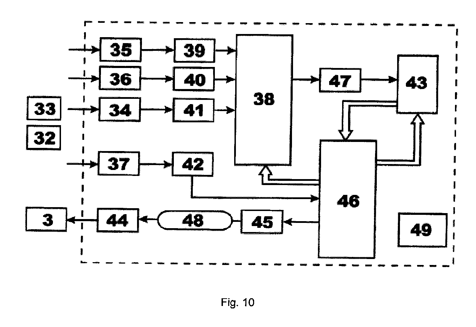

Fig. 10    das Blockschaltbild des Messsignalwandlers.

Die Einrichtung zur Betongefügeanalyse und Betonfestigkeitsmessung und -prüfung besteht aus einer Bohranlage 1, einer Kühlschmiermittel-Rückführungsanlage (Kühlschmiermittel) 2, einer EDV-Anlage (Rechner), einem Personalcomputer 3 mit Controllern und einer elektrischen Messschaltung mit einem Ausgang zum Personalcomputer 3.

Die Bohranlage 1 hat einen Grundkörper 4. Daran sind Führungen 5 senkrecht angebaut und befestigt. Ein Vorschubantrieb 6 ist an den Führungen 5 linear verfahrbar. Der Vorschubantrieb 6 ist zugleich so ausgebildet, dass eine Drehbewegung gleichzeitig an eine Welle 7 übertragbar ist. Die Welle 7 tritt aus dem Vorschubantrieb 6 heraus. Am Auslaufende der Welle 7 sind ein Schneid-Bohrwerkzeug 8 und eine Kühlschmiermittel-Versorgungseinrichtung 9 für ein Schneid-Bohrwerkzeug 8 befestigt. Die Bohranlage 1 ist mit einem Rechtwinkligkeitsregler 10 versehen, um die Bewegung des Schneid-Bohrwerkzeugs 8 senkrecht zum Grundkörper 4 sicherzustellen. Die Bohranlage 1 ist mit Befestigungsmitteln 12 versehen, um den Grundkörper 4 an der zu prüfenden Probe oder am Betonerzeugnis 11 festzumachen. Eine solche Ausführung ermöglicht es, die Bohranlage 1 leicht und einfach zu montieren, genau rechtwinkelige Bohrungen auf waagerechten, senkrechten und geneigten Oberflächen zu bohren, und ist dazu sehr kompakt.

Das Schneid-Bohrwerkzeug 8 ist in Form eines Hohlzylinders ausgebildet. An einer Stirnfläche des Hohlzylinders ist ein Kupplungsstück 13 ausgebildet, um das Schneid-Bohrwerkzeug 8 mit der Welle 7 des Vorschubantriebs 6 zu koppeln. An der anderen Stirnfläche sind Hartmetallschneidplatten 14 angebracht und zwar so, dass während des Bohrens mit einem Solldurchmesser eine zylinderförmige Referenz-Betonprobe 15 entnommen werden kann. Dabei ist die Breite der Hartmetallschneidplatte 14 um wenigstens 1 mm größer als die Wandstärke des Zylinders des Schneid-Bohrwerkzeugs 8. Die Hartmetallschneidplatten 14 sind am Zylinder des Schneid-Bohrwerkzeugs 8 symmetrisch befestigt, um die Messgenauigkeit der Kennwerte zu erhöhen, um Messfehler je nach Körnung (Korngröße) von Schotter und Betonzuschlagstoffen zu vermindern und um einen Leistungsverlust der Bohranlage 1 infolge der Reibung des Schneid-Bohrwerkzeugs 8 an der Seitenfläche des Betonerzeugnisses 11 beim Bohren zu vermeiden.

Die Hartmetallschneidplatten 14 sind am Zylinder des Schneid-Bohrwerkzeugs 8 so angebracht, dass der vorgegebene Innendurchmesser für die Behandlung gebildet werden kann, der dem Außendurchmesser der Referenz-Betonprobe gemäß den Vorschriften von GOST 10180-90 oder gleich 100+0,1 mm oder 150+0,1 mm oder 200+0,1 mm oder 300+0,1 mm ist. Dabei sind die Hartmetallschneidplatten 14 so eingebaut, dass sie je nach Verschleiß ausgetauscht werden können.

Die Kühlschmiermittel-Rückführungsanlage 2 ist in Form eines Dichtbehälters ausgebildet. Sie ist mittels eines Schlauchs 16 mit der Kühlschmiermittelversorgungseinrichtung 9 verbunden, die an der Welle 7 installiert ist, und mit einem Schlauch 17 mit einer Sammelleitung 18 versehen, um das Kühlschmiermittel und den Betonstaub aus dem Bohrbereich zu sammeln und diese in dem abgedichteten Behälter der Kühlschmiermittel-Rückführungsanlage 2 aufzunehmen. Das zur Kühlung benutzte Kühlschmiermittel fließt durch ein Filter, um zur Bohrstelle über ein geschlossenes Kreislaufsystem rückgeführt zu werden. Die Kühlschmiermittel-Rückführungsanlage 2 kann auch unabhängig von einer Wasserleitung betrieben werden und hat ein automatisches Reinigungssystem. Sie lässt sich leicht transportieren und ist dank dem Kühlschmiermittel-Rückumlauf kosteneffizient.

Die Kühlschmiermittelversorgung während des Bohrvorgangs kann mit Hilfe eines Hahns 19 geregelt werden. Dadurch können sowohl Fernwasserleitungen als auch tragbare Behälter zur Kühlung des Schneid-Bohrwerkzeugs 8 eingesetzt werden. Die Sammelleitung 18 zur Sammlung des Kühlschmiermittels hat ein Übergangsstück für den Anschluss an einen Industriesauger (nicht abgebildet) und ermöglicht es, nach Abschluss des Abkühlzyklus praktisch das ganze Kühlschmiermittel zu sammeln. Der Einsatz von Kühlschmiermittel zum Kühlen des Schneid-Bohrwerkzeugs 8 verlängert beachtlich dessen Lebensdauer. Die Sammelleitung 18 zum Sammeln von Kühlschmiermittel sorgt dafür, dass das Sammeln umweltfreundlich ohne Staub und Schmutz durchgeführt wird.

Die EDV-Anlage (Personalcomputer 3) ist so ausgelegt und installiert, dass im Laufe der Prüfung die empfangenen Messdaten erfasst und ausgewertet werden können.

Die elektrische Messschaltung dient dazu, die im Prüfungsprogramm vorgesehenen Kennwerte zu messen und die Messdaten abzulesen und diese nachher zur Weiterverarbeitung an den PC 3 der EDV-Anlage zu übertragen. Sie aktiviert ein Vorschubsteuerungssystem 20 und ein Drehzahlsteuerungssystem 21 des Schneid-Bohrwerkzeugs 8.

Das Vorschubsteuerungssystem 20 enthält eine Vorschubgeschwindigkeitssteuerung 22 und die damit in Reihe geschalteten Vorschubeinrichtung 23 und Werkzeugdrucksensor 24. Der Werkzeugdrucksensor 24 hat eine Rückkopplung mit der Vorschubgeschwindigkeitssteuerung 22.

Das Drehzahlsteuerungssystem 21 des Schneid-Bohrwerkzeugs 8 hat eine Drehzahlsteuerung 25 und eine damit in Reihe geschaltete Drehvorrichtung 26 des Schneid-Bohrwerkzeugs 8 und einen Drehzahlgeber 27, der eine Rückkopplung mit der Drehzahlsteuerung 25 hat.

Die elektrische Messschaltung umfasst den Personalcomputer 3 und den Controller 28, der dem PC 3 vorgeschaltet ist. Am Eingang des Personalcomputers 3 sind die Vorschubgeschwindigkeitssteuerung 22 und die Drehzahlsteuerung 25 angeschlossen. Am Controller 28 sind ein Weggeber 29, ein Spannungsgeber 30 und ein Drehmomentgeber 31 angeschlossen. Beim Einsatz des Drehmomentgebers 31 zwischen dem Antriebsmotor und dem System zur Drehmomentübertragung an das Schneid-Bohrwerkzeug 8 im Leerlauf ermöglicht der Drehmomentgeber 31, die Verlustleistung am Werkzeugantrieb zu ermitteln. Wird der Drehmomentgeber 31 unmittelbar vor dem Schneid-Bohrwerkzeug 8 angeordnet, so werden die mechanischen Verluste automatisch bei der Leistungsermittlung mit berücksichtigt.

Die elektronische Messschaltung der Bohranlage 1 umfasst einen Messsignalwandler 32, der die Bohranlage 1 speist und gleichzeitig die von ihr aufgenommene Leistung ermittelt. Er wird als Messsignalwandler mit einem ungleichmäßigen Aufbau des Abtastfelds zum Messen von Schnellsignalen verwendet, die in Kurzzeitschreibern eingesetzt werden. Die Nutzung eines solchen Wandlers erhöht die Prüfungsleistung.

Der Messsignalwandler 32 aktiviert einen Leichtgängigkeitsregler 33 mit einem Weg-Messwandler 34, einen Strom-Messwandler 35, einen Spannungs-Messwandler 36, einen Drehzahl-Messwandler 37, einen Umschalter 38, Pufferverstärker 39, 40, 41, 42, einen Analog-Digital-Umsetzer 43, die Schnittstelle 44 des Personalcomputers 3 oder die Schnittstelle 45 eines Microcontrollers 46.

Dabei ist jeder Strom-Messwandler 35, Spannungs-Messwandler 36 und Weg-Messwandler 34 jeweils in Reihe mit einem Pufferverstärker geschaltet: Der Strom-Messwandler 35 ist mit dem Pufferverstärker 39, der Spannungs-Messwandler 36 mit dem Pufferverstärker 40 und der Weg-Messwandler 34 mit dem Pufferverstärker 41 in Reihe geschaltet. Der Ausgang jedes Pufferverstärkers 39, 40, 41 ist mit dem Eingang des Analog-Digital-Umschalters 38 verbunden. Der Drehzahl-Messwandler 37 ist mit dem Pufferverstärker 42 in Reihe geschaltet, dessen Ausgang mit dem Eingang des Microcontrollers 46 verbunden ist. Der Ausgang des Umschalters 38 ist mit dem Eingang einer Puffereinrichtung 47 verbunden, deren Ausgang mit dem A-D-Umsetzer 43 verbunden ist. Der Ausgang des Analog-Digital-Umsetzers 43 ist an den zweiten Eingang des Microcontrollers 46 angeschlossen. Ein Ausgang des Microcontrollers 46 ist mit dem zweiten Eingang des Umschalters 38 und der andere Ausgang ist mit dem zweiten Eingang des Analog-Digital-Umsetzers 43 verbunden. Der dritte Ausgang des Microcontrollers 46 ist mittels Schnittstelleneinheiten 44 und 45 über ein Filter 48 mit dem PC 3 in Reihe geschaltet. Der Messsignalwandler 32 aktiviert eine Stromversorgung 49.

Das Verfahren zur Betongefügeanalyse und Betonfestigkeitsprüfung wird wie folgt durchgeführt.

Für die Betongefügeanalyse und Betonfestigkeitsprüfung wird eine Stelle zur Untersuchung einer Betonbaukonstruktion gewählt und für die Prüfungen abgeglichen, z. B. geschliffen mit einem Schneidwerkzeug, z. B. einem Fräser. Bei der Wahl des Werkzeugs zur Vorbereitung der Oberfläche und zwar zum Schleifen oder Glätten von Betonoberflächen oder zur Entfernung von dem vorhergehenden Bearbeitungsmaterial wird Arbeit unter Schwerlast verstanden, die großen Kraftaufwand voraussetzt. Damit wird die zu untersuchende Stelle vorbehandelt. Danach wird an der vorbereiteten Stelle die Oberflächenfestigkeit im Trockenzustand bestimmt. Dann wird diese Stelle eingeweicht und ihre Oberflächenfestigkeit wird nun unter Berücksichtigung ihrer Feuchtigkeit ermittelt. Die Werte werden in den Rechner 3 eingegeben.

Danach wird die Bohranlage 1 auf die zu untersuchende Stelle des Betonerzeugnisses 11 aufgestellt, um die Werte über lagenweises Betongefüge und -nassfestigkeit abzulesen. Die Bohranlage 1 wird mit Hilfe von Befestigungselementen 12 festgemacht. Auf die Bohrstelle wird Kühlschmiermittel aus der Kühlschmiermittel-Rückführungsanlage 2 über die Vorschubvorrichtung 9 an das zylinderförmige hohle Schneid-Bohrwerkzeug 8 zugeführt. Der Bohrvorgang fängt an. Während des Bohrens werden am Bildschirm des Personalcomputers 3 Leistungs-, Weg- und Geschwindigkeitskennlinien erzeugt, welche das Gefüge und die Festigkeit des Betons kennzeichnen.

Das Betonerzeugnis 11 wird in folgender Reihenfolge geprüft:

1. Die Bohranlage 1 wird gestartet. Die Leistungsverluste - Leerlaufleistung - werden je nach der Anordnung des Drehmomentgebers 31 bestimmt.

2. Die Bohrgeschwindigkeit wird eingestellt, indem die Sollgeschwindigkeit als Zahlenwert im PC 3 eingegeben wird.

3. Auswahl des Bohrmodus: Es wird entweder "mit konstantem Solldruck" oder "mit konstanter Sollvorschubgeschwindigkeit" vorgeschoben.

4. Beim Bohrmodus "Vorschub mit konstantem Solldruck" wird im PC 3 der Druck des Schneid-Bohrwerkzeugs 8 auf das Betonerzeugnis 11 vorgegeben. Der Vorschubdruck kann je nach verschiedenen Kennwerten automatisch gewählt werden, z. B. voraussichtliche Betonsorte des Betonerzeugnisses usw. Die Anfangsgeschwindigkeit für die lineare Bewegung des Schneid-Bohrwerkzeugs 8 wird automatisch oder vom Bediener eingegeben.

5. Bei der Maximalgeschwindigkeit wird das Schneid-Bohrwerkzeug 8 vorgeschoben, bis es das Betonerzeugnis 11 berührt.

6. Dann wird auf die Sollgeschwindigkeit für die lineare Bewegung des Schneid-Bohrwerkzeugs 8 beim Bohren umgeschaltet.

7. Die Messwerte des Werkzeugdrucksensors 24 werden von der Vorschubgeschwindigkeitssteuerung 22 abgelesen.

8. Ist der Istwert des Vorschubdrucks größer als der Sollwert, so wird die Vorschubgeschwindigkeit des Schneid-Bohrwerkzeugs 8 verringert.

9. Unterschreitet der Ist-Vorschubdruck den Sollwert, dann wird die Vorschubgeschwindigkeit des Schneid-Bohrwerkzeugs 8 erhöht.

10. Wurde die vorgegebene Bohrtiefe infolge dieser Handlungen nicht erreicht, so werden Schritte 7, 8, 9 wiederholt und zwar: Die Messwerte des Werkzeugdrucksensors 24 werden in die Vorschubgeschwindigkeitssteuerung 22 abgelesen. Überschreitet der Ist-Vorschubdruck den Sollwert, so wird die Vorschubgeschwindigkeit des Schneid-Bohrwerkzeugs 8 vermindert; ist der Ist-Vorschubdruck kleiner als der Sollwert, wird die Vorschubgeschwindigkeit des Schneid-Bohrwerkzeugs 8 erhöht.

11. Beim Erreichen der Soll-Bohrtiefe wird die Bohranlage 1 gestoppt. Das Bohren ist abgeschlossen.

12. Beim Bohrmodus "Vorschub mit konstanter Geschwindigkeit für die lineare Bewegung" wird der konstante Geschwindigkeitssollwert für die lineare Bewegung des Schneid-Bohrwerkzeugs 8 beim Bohrvorgang eingestellt. Die Geschwindigkeit für die lineare Bewegung kann automatisch gewählt werden.

13. Es werden die Schritte 5 und 6 ausgeführt und zwar: Das Schneid-Bohrwerkzeug 8 wird mit Maximalgeschwindigkeit vorgeschoben, bis es das Betonerzeugnis 11 kontaktiert. Dann wird auf die Sollgeschwindigkeit für die lineare Bewegung des Schneid-Bohrwerkzeugs 8 beim Bohren umgeschaltet.

14. Der lineare Verfahrweg des Schneid-Bohrwerkzeugs 8 innerhalb einer Zeitspanne wird in den Controller 28 abgelesen und an den PC 3 übertragen. Der Personalcomputer 3 berechnet die lineare Geschwindigkeit des Schneid-Bohrwerkzeugs 8.

15. Ist die lineare Ist-Verfahrgeschwindigkeit kleiner als der Sollwert, dann wird die Vorschubgeschwindigkeit erhöht.

16. Überschreitet die lineare Ist-Verfahrgeschwindigkeit den Sollwert, wird die Vorschubgeschwindigkeit vermindert.

17. Wurde die vorgegebene Bohrtiefe infolge dieser Handlungen nicht erreicht, so werden die Schritte 14, 15, 16 wiederholt und zwar: Der Ist-Wert der linearen Verfahrgeschwindigkeit des Schneid-Bohrwerkzeugs 8 innerhalb einer Zeitspanne wird in den Controller 28 abgelesen und an den PC 3 gesendet. Der PC 3 berechnet die lineare Verfahrgeschwindigkeit des Schneid-Bohrwerkzeugs 8. Unterschreitet die lineare Verfahrgeschwindigkeit den Sollwert, wird die Vorschubgeschwindigkeit erhöht; ist die lineare Verfahrgeschwindigkeit größer als Sollwert, wird sie reduziert.

18. Ist die vorgegebene Bohrtiefe erreicht, wird die Bohranlage 1 gestoppt. Das Bohren ist abgeschlossen.

Bei der Ausführung der Schritte 6 - 10 und 12 - 17 werden die Daten von Drehmomentgeber 31, Weggeber 29 und Speisespannungsgeber 30 ununterbrochen in den Controller 28 abgelesen und an den PC 3 übertragen. Gleichzeitig werden die Vorschubdruckwerte aus der Vorschubgeschwindigkeitssteuerung 22 und die Drehzahlwerte aus der Drehzahlsteuerung 25 abgelesen und an den PC 3 gesendet. Anhand der empfangenen Daten wird die Leistung berechnet. Im PC 3 werden die Geschwindigkeit der linearen Bewegung des Schneid-Bohrwerkzeugs 8 und die für das Bohren verbrauchte Energie ermittelt. Anschließend werden diese Daten in Form von Kennlinien und Zahlenwerten auf dem Bildschirm ausgegeben.

Der Rechner 3 steuert den Betrieb der Einrichtung zur Betongefügeanalyse und die Betonfestigkeitsmessung und -prüfung und ermöglicht es, sowohl den gesamten Bohr- und Auswertungsvorgang als auch nur einen beliebigen vom Bediener gewählten Abschnitt der Prüfung grafisch darzustellen und auszudrucken. Während des Bohrens kann der Rechner 3 die Erstellung der Ausgabedatei unterbrechen. Darüber hinaus kann der Rechner 3 während der Verarbeitung der Eingangsdaten einen beliebigen Zeitabschnitt aus der Berechnung löschen, wobei die Kontinuität der Bewegung erhalten bleibt.

Beim Laden des Arbeitsprogramms und seiner richtigen Einstellung werden beim angeschlossenen Controller die ermittelte Leistung, der Weg, die Drehzahl des Schneid-Bohrwerkzeugs 8, die Speisespannung und die Anzahl der vom Controller empfangenden Datenbytes angezeigt. Um die Erstellung der Ausgabedatei anzufangen, muss ein Befehl abgegeben werden. Danach fängt die Ermittlung der Leerlaufgeschwindigkeit an, das heißt die Ermittlung eines solchen Wertes, der nachher von der Gesamtleistung während des Bohrvorgangs abgezogen wird. Während des Bohrens wird die Kennlinie der aktuellen Leistung unter Abzug der Leerlaufleistung und der Wegleistung erstellt.

Bei der Rohdatenverarbeitung werden folgende Aufgaben gelöst:

- Löschen des nicht aussagekräftigen Bohrabschnitts aus dem Verarbeitungsumfang;
- Wiederherstellung der Kontinuität der Wegwerte;
- Ermittlung der zunehmenden Wegwerte;
- Ermittlung der mit "0" beginnenden Wegwerte; diese werden dabei als gleich 0 betrachtet, und alle übrigen Werte werden additiv korrigiert;
- Korrektur der Messdaten in Bezug auf die Leistung und den Weg.

Nach Abschluss der Arbeit mit der Rohdatendatei wird das Verarbeitungsergebnis abgespeichert. Damit ist die primäre Bearbeitung der Rohdatendatei abgeschlossen.

Um nach der Bearbeitung ansteigende Wegwerte zu haben, werden nach folgendem Algorithmus invertiert:

$$dl = L(1) + L(N), \quad L(i) = dl - L(i),$$

wobei
$L(1)$ und $L(N)$ die Wegwerte im 1. und im letzten Punkt sind.

Unabhängig vom Maßstab der Kennlinie wird die ganze Datei verarbeitet.

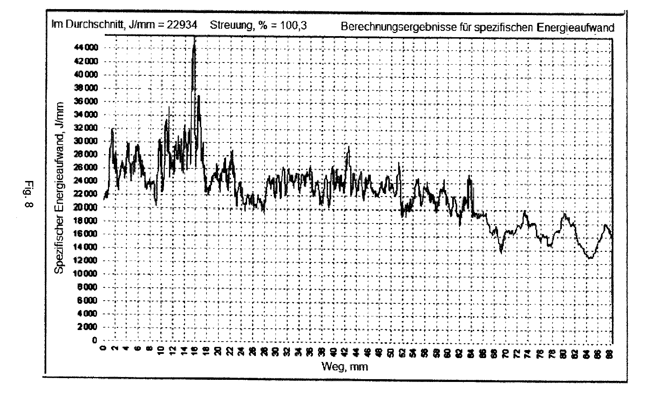

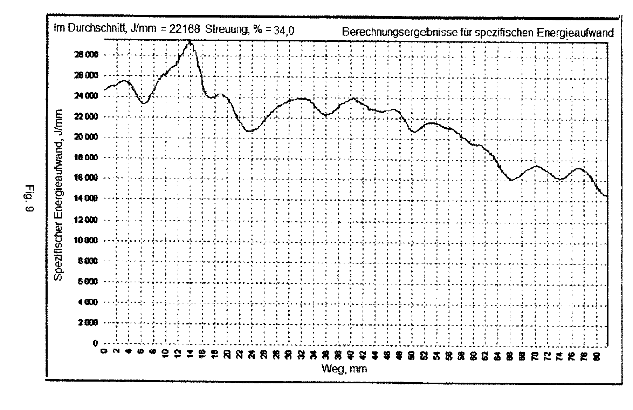

Danach wird der spezifische Energieaufwand berechnet. Die ermittelten Daten werden bearbeitet, um den Energieaufwand für das Bohren von einem Millimeter Material in J/mm zu bestimmen. Die Wegwerte werden bei der Kennlinie aus Fig. 8 in der X-Achse und der Energieaufwand wird in der Y-Achse abgegriffen. Dabei kann das Intervall für die berechneten Kenngrößen verändert werden. Dafür muss der Intervallwert eingegeben werden. Der Mindestwert für ein Intervall ist 5, der Maximalwert wird nicht begrenzt. Beim größeren Intervallwert liegt mehr ausgeprägte Abflachung der Kennlinie vor, s. Fig. 9.

Die Berechnungsergebnisse können in einer Datei mit der Endung ".rez" abgelegt werden. In der Kennlinie aus Fig. 8 sind der Mittelwert für den spezifischen Energieaufwand sowie die Datenstreuung dargestellt. Dabei wird jenes Wegintervall berücksichtigt, welches momentan angezeigt wird.

Nach Abschluss des Bohrvorgangs und infolge des Bohrens mit einem zylinderförmigen hohlen Schneid-Bohrwerkzeug 8 wird zusätzlich eine zylinderförmige Referenz-Betonprobe 15 erzeugt. Die zylinderförmige Referenz-Betonprobe 15 wird einer zusätzlichen Prüfung unter Laborbedingungen ausgesetzt, wobei die Druck- oder axiale Zugfestigkeit der Referenz-Betonprobe gemäß den Vorschriften von GOST 18105-86, GOST 10180-90 bestimmt wird. Diese Festigkeit wird im Rechner 3 ausgewertet. Dabei werden diese Angaben mit den vorherigen anhand anderer Verfahren ermittelten Angaben verglichen. Die zylinderförmige Referenz-Betonprobe 15 wird getrocknet. Das beste Ergebnis wird mittels Spülbohrens erreicht, weil das Kühlschmiermittel das Schneid-Bohrwerkzeug 8 abkühlt und das Bohrmehl entfernt. Somit wird der Verschleiß des zylinderförmigen Schneid-Bohrwerkzeuges 8 minimiert.

Der Einsatz der vorgeschlagenen technischen Lösung ermöglichte es, ein Verfahren zu entwickeln, mit dessen Hilfe die Genauigkeit der Betongefügeanalyse und der lagenweisen Betonfestigkeitsprüfung erhöht wurde. Die entwickelte Einrichtung für die Ausführung dieses Verfahrens ermöglicht es, genaue Ausgabedaten für die Betongefügeanalyse des Erzeugnisses zu bekommen und zufriedenstellende Genauigkeit der Betonfestigkeitsmessung und -prüfung des Erzeugnisses dank der Vorbehandlung der zu untersuchenden Oberfläche zu erreichen. Darüber hinaus wurde gemäß der vorgeschlagenen technischen Lösung eine Einrichtung hergestellt, um beim Bohren gleichzeitig eine zylinderförmige Referenz-Betonprobe zusätzlich zu entnehmen. Diese Referenz-Betonprobe wird nachher für weitere Prüfungen von Druck- oder axialer Zugfestigkeit unter Laborbedingungen verwendet. Darüber hinaus ist das vorgeschlagene Verfahren durch minimalen Arbeitsaufwand und minimale Kosten gekennzeichnet. Außerdem ermöglicht es die vorgeschlagene technische Lösung, die Lagenfestigkeit sogar beim Bohren von einem stark bewehrten Betonerzeugnis zu kontrollieren und ideal ebene (gerade) Bohrlöcher mit den genau eingehaltenen normengerechten Durchmessern zu bekommen. Dabei entsteht während des Bohrvorgangs überhaupt kein Betonstaub, der seit langem als eine der gesundheitsschädlichsten Substanzen gilt. Das macht diesen Prüfungsprozess zu einem der umweltfreundlichsten unter allen möglichen Verfahren in dieser Branche. Darüber hinaus werden eine hohe Arbeitsgenauigkeit der Bohranlage, Sauberkeit und relative Geräuscharmut erreicht. Die Bohranlage erzeugt beim Betrieb so gut wie keine Vibrationen des Werkzeugs. Deswegen liegt keine Gefahr von Rissbildung in den empfindlichen Stellen des Betonerzeugnisses vor, und es wird eine niedrige Ausgangsleistung vorausgesetzt.

**Patentansprüche**

1. Verfahren zur Betongefügeanalyse und Betonfestigkeitsprüfung bei Bauteilen einschließlich folgender Schritte: Einweichung von Beton, Bohren von Beton, Ermittlung von Bohrleistung, Messung der Größe und der Geschwindigkeit des Bohrwerkzeug-vorschubs mit einer Datenausgabe in Form von Leistungs-, Weg- und Geschwindigkeitskennlinien des Bohrwerkzeugs, welche das Gefüge und die lagenweise Festigkeit des Betons kennzeichnen, wobei die zahlenmä-βigen Angaben für jede Kennlinie mit ausgegeben werden,
   **dadurch gekennzeichnet,**
   **dass** die Oberfläche der zu untersuchenden Stelle des für die Betongefügeanalyse und die Betonfestigkeitsprüfung gewählten Betonerzeugnisses vor der Prüfung vorbereitet wird, indem die zu untersuchende Stelle geschliffen wird,
   **dass** zuerst ihre Oberflächenfestigkeit im Trockenzustand ermittelt wird,
   **dass** dann dieser Bereich der zu untersuchenden Oberfläche des Betonerzeugnisses eingeweicht und die Oberflächenfestigkeit des Betons unter Beachtung seiner Feuchtigkeit ermittelt wird,
   **dass** danach eine Bohranlage zum Betonausbohren auf die zu untersuchende Stelle aufgestellt wird und mittels Bohrens das lagenweise Gefüge und die lagenweise Nassfestigkeit des Betons ermittelt werden,
   **dass** infolge des Bohrvorgangs zusätzlich eine zylinderförmige Referenz-Betonprobe entnommen wird, welche für weitere Prüfungen bei der Ermittlung von Druck- oder axialer Zugfestigkeit der Referenz-Betonprobe verwendet wird und
   **dass** dabei die Angaben mit vorherigen anhand anderer Verfahren ermittelten Angaben verglichen werden und die Referenz-Betonprobe vorher getrocknet wird.

2. Verfahren nach Anspruch 1,
   **dadurch gekennzeichnet,**
   **dass** am Anfang des Bohrens, um den Datenempfangsablauf zu steuern, die aktuellen Einstellungen der Geräte angezeigt werden,
   **dass** während des Bohrens Momentanmessdaten der Kennwerte empfangen und über eine Datenübertragungsschnittstelle an einen PC oder Microcontroller zur Verarbeitung und Berechnung von Ergebnissen über den spezifischen Energieaufwand pro Vorschublängeneinheit des Schneid-Bohrwerkzeugs beim Bohren des Betonerzeugnisses gesendet werden und
   **dass** dabei eine Leistungskennlinie und eine Vorschubkennlinie des Schneid-Bohrwerkzeugs erstellt werden.

3. Verfahren nach Anspruch 1,
   **dadurch gekennzeichnet,**
   **dass** die zylinderförmige Betonprobe für weitere Prüfungen mit Hilfe eines zylinderförmigen Hohl-Schneid-Bohrwerkzeugs zum Bohren von Betonerzeugnissen entnommen wird.

**4.** Einrichtung zur Betongefügeanalyse und Betonfestigkeitsprüfung bei Bauteilen mit einer Bohranlage, welche einen Vorschubantrieb für eine lineare Bewegung und Drehung des Schneid-Bohrwerkzeugs aufweist, einem Oberflächenbetonfestigkeits-Messgerät, einem Weggeber des Bohrwerkzeugs, Leistungsgebern zur Messung der vom Antrieb aufgenommenen Leistung und einem Controller mit einem Rechner,
**dadurch gekennzeichnet,**
**dass** die Bohranlage mit

- einem Rechtwinkligkeitsregler für die rechtwinklige Bewegung des Schneid-Bohrwerkzeugs,
- einem Druckgleichhalter, um den auf das Schneid-Bohrwerkzeug ausgeübten Druck gleich zu halten,
- einem Messsignalwandler,
- einer Kühlschmiermittel-Rückführungsanlage versehen ist und

**dass** das Schneid-Bohrwerkzeug in Form eines Hohlzylinders ausgebildet ist, wobei an einer Stirnfläche des Hohlzylinders ein Kupplungsstück zum Anschluss des Hohlzylinders an den Vorschubantrieb und eine Kühlschmiermittel-Versorgungseinrichtung angebracht ist und an der anderen Stirnfläche Hartmetallschneidplatten befestigt sind.

**5.** Einrichtung nach Anspruch 4,
**dadurch gekennzeichnet,**
**dass** Hartmetallschneidplatten des Schneid-Bohrwerkzeuges so eingebaut sind, dass während des Bohrens eine zylinderförmige Referenz-Betonprobe mit einem Solldurchmesser entnehmbar ist,
**dass** jede Hartmetallschneidplatte dabei am Zylinder des Schneid-Bohrwerkzeugs symmetrisch befestigt ist, um die Messgenauigkeit der Kennwerte zu erhöhen, indem die Messfehler je nach Körnung von Schotter und Betonzuschlagstoffen vermindert sind und ein Leistungsverlust der Bohranlage infolge der Reibung des Schneid-Bohrwerkzeugs an der Seitenfläche des Betonerzeugnisses vermieden ist und
**dass** die Breite jeder Hartmetallschneidplatte wenigstens um 1 mm größer als die Wandstärke des Zylinders des Schneid-Bohrwerkzeugs ist.

**6.** Einrichtung nach Anspruch 4,
**dadurch gekennzeichnet,**
**dass** die Hartmetallschneidplatten am Zylinder des Schneid-Bohrwerkzeugs so angebracht sind, dass der vorgegebene Innendurchmesser für die Behandlung gebildet ist, der entweder gleich 100+0,1 mm oder 150+0,1 mm oder 200+0,1 mm oder 300+0,1 mm ist.

**7.** Einrichtung nach Anspruch 4,

**dadurch gekennzeichnet,**
**dass** Hartmetallschneidplatten so eingebaut sind, dass sie je nach Verschleiß austauschbar sind.

**8.** Einrichtung nach Anspruch 4,
**dadurch gekennzeichnet,**
**dass** der Messsignalwandler mit einem Leichtgängigkeitsregler mit Weg-Messwandler, einem Strom-Messwandler, einem Spannungs-Messwandler, einem Drehzahl-Messwandler, einem Umschalter, Schnittstelleneinheiten, Pufferverstärkern, einem A-D-Umsetzer, einer PC- oder Microcontroller-Schnittstelle ausgestattet ist,
**dass** der Strom-Messwandler, der Spannungs-Messwandler und der Weg-Messwandler dabei jeweils mit je einem Pufferverstärker in Reihe geschaltet sind, wobei der Ausgang jedes Pufferverstärkers mit dem Eingang eines Umschalters verbunden ist,
**dass** der Drehzahl-Messwandler mit dem Pufferverstärker in Reihe geschaltet ist, dessen Ausgang mit dem Eingang des Microcontrollers verbunden ist, dass der Ausgang des Umschalters an den Eingang der Puffereinrichtung angeschlossen ist, deren Ausgang mit dem A-D-Umsetzer verbunden ist, dessen Ausgang an den zweiten Eingang des Microcontrollers angeschlossen ist, dass ein Ausgang des Microcontrollers an den zweiten Eingang des Umschalters und der andere Ausgang an den zweiten Eingang des Analog-Digital-Umsetzers angeschlossen ist, und
**dass** der dritte Ausgang des Microcontrollers mittels Schnittstelleneinheiten über ein Filter mit dem PC in Reihe geschaltet ist.

Fig. 1

Fig. 2

A-A

Fig. 3

$t_Z$

$>0,5\,mm$

$t_R > t_Z$

Fig. 4

Fig. 5

Fig. 6

EP 2 573 557 A1

Fig. 7

15

Fig. 8

Fig. 9

Fig. 10

## INTERNATIONAL SEARCH REPORT

| International application No. |
| --- |
| PCT/RU 2011/000112 |

**A. CLASSIFICATION OF SUBJECT MATTER**

G01N 33/38 (2006.01)  G01N 3/00 (2006.01)

According to International Patent Classification (IPC) or to both national classification and IPC

**B. FIELDS SEARCHED**

Minimum documentation searched (classification system followed by classification symbols)

G01N 33/38, 3/00

Documentation searched other than minimum documentation to the extent that such documents are included in the fields searched

Electronic data base consulted during the international search (name of data base and, where practicable, search terms used)

RUPAT, USPAT, WIPO, Esp@cenet, PatSearch

**C. DOCUMENTS CONSIDERED TO BE RELEVANT**

| Category* | Citation of document, with indication, where appropriate, of the relevant passages | Relevant to claim No. |
| --- | --- | --- |
| A | RU 2179722 C2 (SAMARSKAYA GOSUDARSTVENNAYA ARKHITEKTURNO-STROILTELNAYA AKADEMIYA) 20.02.2002 | 1-8 |
| A | RU 2198399 C2 (SAMARSKAYA GOSUDARSTVENNAYA ARKHITEKTURNO-STROILTELNAYA AKADEMIYA) 10.02.2003 | 1-8 |
| A | JP 2001004512 A (TAKENAKA KOMUTEN CO) 12.01.2001 (the abstract) | 1-8 |
| A | JP 59137839 A (NIPPON STEEL CORP) 08.08.1984 (the abstract) | 1-8 |
| A | JP 2008128831 A (OHBAYASHI CORP) 05.06.2008 (the abstract) | 1-8 |

☐ Further documents are listed in the continuation of Box C.   ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 20 July 2011 (20.07.2011) | 18 August 2011 (18.08.2011) |

| Name and mailing address of the ISA/ | Authorized officer |
| --- | --- |
| | |
| Facsimile No. | Telephone No. |

Form PCT/ISA/210 (second sheet) (July 1998)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- RU 2235322
- RU 3452179722

- RU 2198399